(19) Europäisches Patentamt
European Patent Office
Office européen des brevets

(11) **EP 2 612 688 B1**

(12) **EUROPÄISCHE PATENTSCHRIFT**

(45) Veröffentlichungstag und Bekanntmachung des Hinweises auf die Patenterteilung:
**14.03.2018   Patentblatt 2018/11**

(51) Int Cl.:
*A61M 16/00* *(2006.01)*          *H02J 7/14* *(2006.01)*

(21) Anmeldenummer: **13000070.6**

(22) Anmeldetag: **08.01.2013**

(54) **Vorrichtung zur Beatmung**

Device for breathing

Dispositif d'assistance respiratoire

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.01.2012   DE 102012000192**

(43) Veröffentlichungstag der Anmeldung:
**10.07.2013   Patentblatt 2013/28**

(73) Patentinhaber: **Weinmann Emergency Medical Technology GmbH + Co. KG**
**22525 Hamburg (DE)**

(72) Erfinder:
• **Schwalbe, Billy**
**22159 Hamburg (DE)**
• **Herrmann, Frank**
**25355 Barmstedt (DE)**

(74) Vertreter: **Klickow & Wetzel PartGmbB**
**Jessenstraße 4**
**22767 Hamburg (DE)**

(56) Entgegenhaltungen:
EP-A1- 1 366 948          EP-A1- 1 575 153
EP-A2- 0 923 183          WO-A1-00/27021
DE-A1- 19 522 563         US-A1- 2010 170 513
US-A1- 2011 193 410

**Beschreibung**

Die Erfindung betrifft ein Beatmungsgerät mit einem Elektromotor und zumindest einer primären Energiequelle für den Elektromotor und einer sekundären Energiequelle, wobei die sekundäre Energiequelle als Spannungssenke für Generatorstrom vom Elektromotor ausgebildet ist.

Gebläsebetriebene Beatmungsgeräte passen den Beatmungsdruck durch Variation der Drehzahl eines Elektromotors an. Der erzeugte Atemgas-Druck ist dabei etwa proportional zum Quadrat der Drehzahl. Um unter typischen Beatmungsbedingungen den Druck anzupassen, müssen Drehzahlvariationen von vielen tausend Umdrehungen pro Minute realisiert werden. Dazu wird die Rotationsenergie der sich drehenden Komponenten verändert, so dass in der Inspirationsphase

Rotationsenergie ins System gesteckt werden muss, die in der Exspirationsphase dem System zu entziehen ist. Diese Energie wird üblicherweise in Wärme umgesetzt.

Zum Wechsel zwischen Beschleunigen und Bremsen werden normalerweise unterschiedliche Regler eingesetzt, weshalb bei Regelschwingungen immer wieder gebremst und hierdurch zusätzliche Energie vernichtet wird. Macht man das Umschalten zwischen den Reglern weniger sensitiv, verschlechtert sich die Druckkonstanz der Beatmung.

Um die Drehzahl eines Gebläses zu verändern, muss die Versorgungsspannung verändert werden. Variiert der Drehmomentbedarf beim Beschleunigen oder Bremsen, so muss der Motorstrom angepasst werden. Hierzu ist zwischen der Spannungsquelle und dem Gebläse ein Spannungs-/Stromwandler einzusetzen.

Das Beatmungsgerät wird über ein entsprechendes Netzteil an das Versorgungsnetz (ortsübliches Wechselspannungsnetz, z.B. in Deutschland 230V 50Hz) angeschlossen. Das Netzteil ist entweder extern oder in das Beatmungsgerät integriert. Die interne Betriebsspannung beträgt je nach Gerät zwischen 12V und 48V. Ist das Gerät mit einem Akku ausgestattet, ist seine Nennspannung in demselben Bereich angeordnet. Die Spannungsversorgung der Elektronik erfolgt stets vom höheren Potential zum niedrigeren.

Die bislang bekannten gebläsebetriebenen Beatmungsgeräte verbrauchen zu viel Strom und erzeugen zu viel Abwärme.

Aus der US 2010/170513 A1 ist es bereits bekannt, den Gebläsemotor eines Beatmungsgerätes wahlweise von einer ersten Energieversorgung oder einer zweiten Energieversorgung zu speisen. Die erste Energieversorgung kann als ein Netzteil und die zweite Energieversorgung als ein Akkumulator ausgebildet sein. Eine Ladung des Akkumulators kann unter Verwendung eines Generators und einer Turbine erfolgen, wobei die vom Patienten bei einer Ausatmung bereitgestellte Druckenergie in elektrische Leistung transformiert wird.

Aufgabe der vorliegenden Erfindung ist es daher, eine Vorrichtung der einleitend genannten Art derart zu konstruieren, dass Stromspitzen reduziert werden und/oder eine Rückgewinnung der Bremsenergie gewährleistet wird.

Diese Aufgabe wird erfindungsgemäß dadurch gelöst, dass der Generatorstrom durch eine Transformation von kinetischer Energie des Elektromotors erzeugt wird, dass die Transformation nach einem Ende einer Inspirationsphase und vor einem Beginn einer Exspiration erfolgt und dass eine Sensoreinrichtung / Analyseeinheit zur Steuerung und Regelung des Beatmungsbetriebes verwendet ist, wobei zur Erfassung eines Druckes ein Druckmessschlauch verwendet ist und wobei der Betrieb der Elektromotors über eine Motorsteuerung regelbar ist und wobei die Sensoreinrichtung mindestens ein mit einem Atemgasstrom im Zusammenhang stehende Signal ermittelt und der Analysator aus diesem Signal die Inspirationsphasen und die Expirationsphasen erkennt, wobei durch eine Drehzahländerung des Elektromotors der Druck einstellbar ist.

Mit Hilfe der Energierückgewinnung soll die absolut benötigte Energie aus der primären Energiequelle des Beatmungsgeräts reduziert werden. Dies führt bei einem akkubetriebenen Gerät zu einer verlängerten Gerätelaufzeit. Ebenso kann die Baugröße des Beatmungsgeräts bei gleichbleibender Laufzeit verkleinert werden.

Eine weitere Aufgabe besteht darin, das Beatmungsgerät für den Einsatz an einem KFZ-Bordnetz und/oder einem Akku zu optimieren. Dabei gilt es, Stromspitzen zu minimieren und das zu entwickelnde Konzept gegenüber dem Stand der Technik nicht zu stark zu verkomplizieren.

Diese Aufgabe wird erfindungsgemäß dadurch gelöst, dass die Bremsenergie in einer sekundären Energiequelle, beispielsweise einem Kondensator oder Akkumulator, gespeichert wird und diese für die nächste Beschleunigungsphase wieder verwendet wird.

Das erfindungsgemäße Beatmungsgerät mit einem Elektromotor und zumindest einer primären Energiequelle für den Elektromotor und einer sekundären Energiequelle ist dadurch gekennzeichnet, dass die sekundäre Energiequelle als Spannungssenke für Generatorstrom vom Elektromotor ausgebildet ist.

Das Beatmungsgerät ist auch dadurch gekennzeichnet, dass die primäre Energiequelle beispielsweise als Netzstecker mit Steckdose oder als Bordnetz eines Fahrzeuges oder als Akkumulator ausgeführt ist.

Die Erfindung betrifft auch ein Verfahren zur Nutzung von Bremsenergie bei einem Beatmungsgerät, wobei der Generatorstrom durch eine Transformation von kinetischer Energie des Elektromotors erzeugt wird und die Transformation nach einem Ende einer Inspirationsphase und vor einem Beginn einer Exspiration erfolgt. Das Verfahren ist auch dadurch gekennzeichnet, dass ein bidirektionaler Stromfluss zwischen Motor und der Energiequelle durch Verwendung zumindest eines Wandlers realisiert ist.

Das Verfahren ist auch dadurch gekennzeichnet, dass das Übertragungsverhältnis von Strom- oder Spannungsfluss zwischen Motor und der Energiequelle durch Pulsweitenmodulation oder Pulsfrequenzmodulation der Leistungsschalter des Wandlers erfolgt.

Das Verfahren ist auch dadurch gekennzeichnet, dass der Elektro-motor im Generatorbetrieb Spannung erzeugt, die in einem Kondensator oder Akkumulator gespeichert wird und die Spannung danach zumindest teilweise wieder für die Energieversorgung des Elektromotors genutzt wird.

Dafür ist der Weg zwischen der Spannungsquelle und dem Gebläse über einen bidirektionalen DC/DC-Synchron-Wandler bidirektional aufgebaut.

Eine typische Ausführungsform der Erfindung findet ihre Anwendung bei der CPAP-Beatmung, Bilevel-Beatmung, Heimbeatmung oder der Notfallbeatmung.

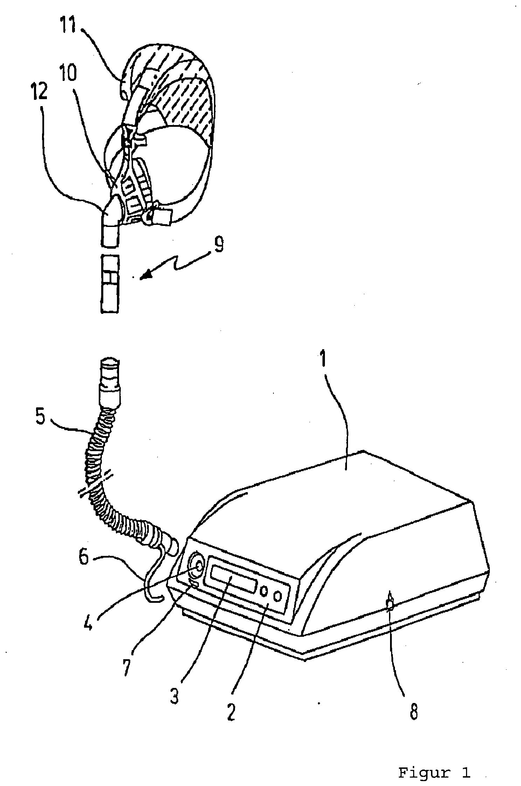

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum ein Elektromotor (13, nicht dargestellt) mit Gebläse angeordnet. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf. Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

Der Motor (13) ist als ein mehrphasiger Motor realisiert. Als Motor können bürsten- und sensorlose Gleichstrommotoren sowie Synchronmotoren verwendet werden. Im Bereich des Gerätegehäuses (1) befindet sich ein Bedienfeld für die Anwenderinformation und / oder Anwendersteuerung (2, 3). Bevorzugt ist das Beatmungsgerät tragbar und verfügt optional über eine Energieversorgung, die die Beatmung zumindest für mehr als 2 Stunden aufrecht erhält, wenn das Gerät nicht über einen Netzstecker mit Energie versorgt wird. Die Mobilität ist durch die zusätzliche Anschlussmöglichkeit an das Bordnetz eines Fahrzeugs (über 12 oder 24 Volt) oder an eine entsprechende andere Energieversorgung erweiterbar.

In einem Geräteinnenraum ist eine Atemgaspumpe angeordnet, die als Elektro-Motor mit Lüfterrad (13) ausgeführt ist, dessen Betrieb über eine Motorsteuerung regelbar ist. Der Betrieb des Motors und dessen Leistungsregelung sind durch die Motorsteuerung regelbar. Die Motorsteuerung berücksichtigt Daten von zumindest einer Sensoreinrichtung. Die Sensoreinrichtung ermittelt zumindest ein mit dem Atemgasstrom in Zusammenhang stehendes Signal. Ein Analysator ermittelt aus dem mit dem Atemgasstrom in Zusammenhang stehenden Signal Inspirationsphasen und Exspirationsphasen. In zumindest einem Betriebszustand regelt die Motorsteuerung die Lüfterraddrehzahl in Abhängigkeit der ermittelten Atemphase derart, dass während der Inspirationsphase ein im Wesentlichen konstanter positiven Druck aufrecht erhalten wird. Der Motor ist derart ausgelegt, dass durch Drehzahländerung ein Druckbereich von im Wesentlichen 0 bis 80 mbar einstellbar ist. Druckänderungen werden durch Drehzahländerungen des Lüfterrades realisiert.

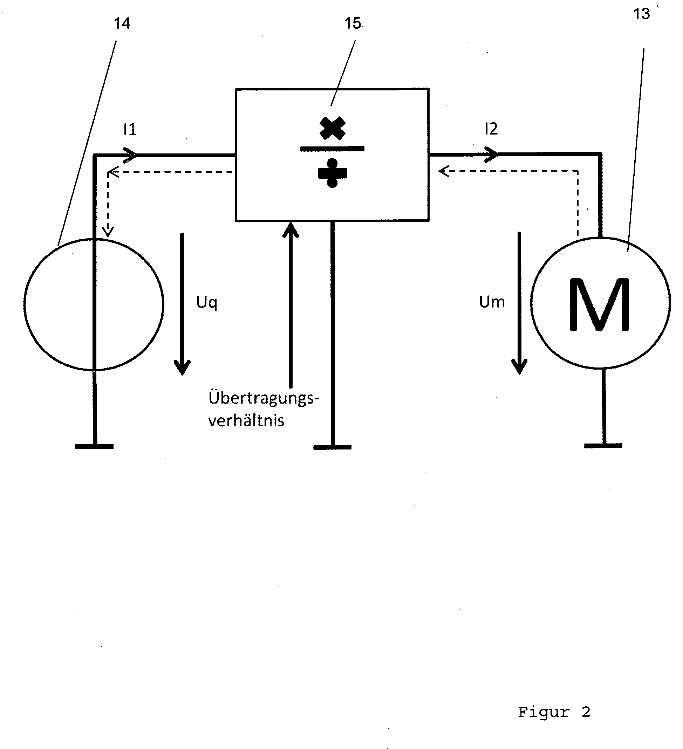

Figur 2 zeigt einen schematischen Schaltaufbau.

Elektromotoren (13), bieten die Möglichkeit der bidirektionalen Stromflussrichtung. Ein Motor kann sowohl als Senke betrachtet werden, wenn er angetrieben und beschleunigt wird, oder auch als Quelle, wenn er im Generatorbetrieb läuft und Strom erzeugt.

Bei einer Läuferdrehzahl des Motors von $n = 0$ 1/min ist der Strom $I = 0$ A. Durch Erhöhen der Quellspannung $Uq$ wird ein positiver Strom $I$ hervorgerufen. Der Strom wird im Motor in Drehmoment $M$ umgewandelt, welches selbigen zum Anlaufen bewegt. Die Drehzahl $n$ steigt. Die im Motor induzierte Gegenspannung $U\_EMK$ steigt proportional zu $n$. Der Strom $I$ verkleinert sich, bis sich der zur Erzeugung des anliegenden Lastdrehmoments $M\_L$ erforderliche Strom $I\_L$ einstellt. Bei gleichbleibendem Drehmoment geht das System in einen stationären Zustand über. Es gilt

$$Uq > U\_EMK$$

$$I\_L;\ Uq;\ n;\ M\_L = konst$$

Ein aktiver Bremsvorgang findet statt, sobald die an den Motor angelegte Klemmspannung $Uq$ kleiner wird als $U\_EMK$. Der resultierende Strom $-I$ erzeugt ein Drehmoment $-M$. Ab diesem Moment arbeitet der Motor als Generator, er erzeugt Strom aus seiner überschüssigen Rotationsenergie. Es fließt ein negativer Strom, solange

$$Uq\ <\ U\_EMK$$

ist. Ist

$$Uq\ =\ U\_EMK$$

wird die Schwelle zwischen Generator und Motorbetrieb überschritten und der Strom beginnt bei Uq > U_EMK wieder in den Motor zu fließen. Es stellt sich ein stationärer Betrieb ein. Die beim Drehzahlabbau freigesetzte Energie wurde in die ursprüngliche Quelle (14) zurückgespeist.

Die zuvor ideal betrachtete Spannungsquelle muss in ihrer Ausgangsspannung veränderlich sein und einen bidirektionalen Stromfluss zulassen, um aktiv mit Energierückgewinnung Bremsen zu können. Figur 2 zeigt die Verwendung eine Spannungsquelle (14) mit fester Ausgangsspannung, bei der zur Spannungsanpassung des Motors eine Transformationsschaltung (15) verwendet wird. Die Schaltung (15) muss je nach eingestelltem Übertagungsverhältnis K wie ein Teiler oder Multiplikator auf Ein- bzw. Ausgangsspannung wirken.

$$Uq\ =\ Um\ *\ K$$

Ebenso wirkt die Schaltung auf die Ströme I1 und I2 mit dem Verhältnis

$$I1\ =\ I2\ /\ K$$

Idealerweise gilt ein Wirkungsgrad von 100% und somit

$$P1\ =\ P2\ =\ Uq\ *\ I1\ =\ Um\ *\ I2$$

Durch die Transformationsschaltung (15) ist es nun möglich, aus einer beliebigen Quellspannung Uq eine beliebige Motorspannung Um einzustellen. Ebenso kann die Stromflussrichtung bei Uq > 0 oder Um > 0 alleine durch den Übertragungsfaktor K eingestellt werden.

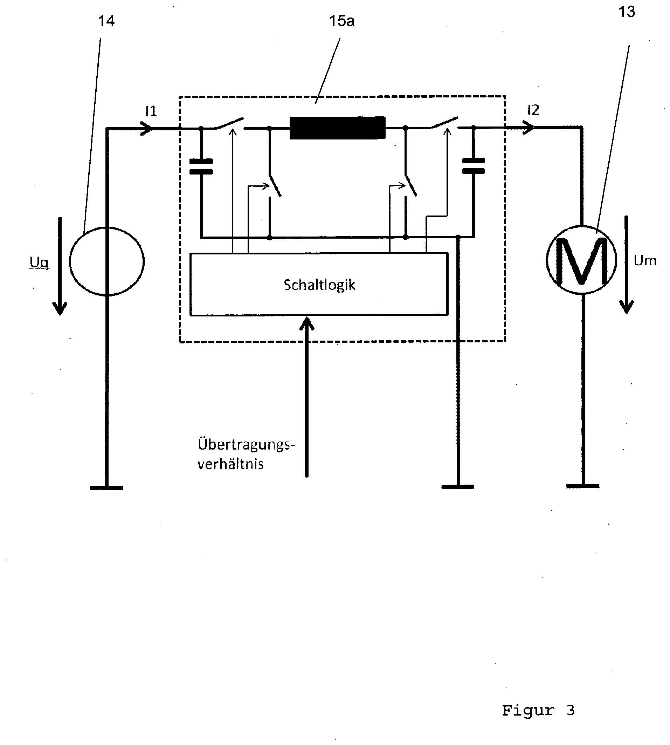

Die in Figur 2 dargestellte Transformationsschaltung (15) kann durch einen Synchronen Auf- und Abwärtswandler (15a) realisiert werden, der in Figur 3 beispielhaft dargestellt ist. Der Übertragungsfaktor K kann beim Synchronwandler durch das Tastverhältnis p, der auf die Leistungsschalter gegebenen Pulsweitenmodulation (PWM) eingestellt werden. Die eingezeichnete Schaltlogik nimmt das Übertragungsverhältnis entgegen und erzeugt daraus eine PWM. Diese wird auf die entsprechenden Transistoren des Schaltwandlers geleitet. Während des Betriebs ist jeweils eine Seite des Synchronen Auf- und Abwärtswandlers (15a) auf Durchgang geschaltet, während die andere Seite durch die PWM getaktet wird. Es ergeben sich zwei Betriebszustände, die vom eingestellten Übertragungsfaktor abhängig sind. Für $K \leq 1$ gilt $Uq \leq Um$ und der linke Zweig des Wandlers muss getaktet werden, während der rechte durchgeschaltet ist. Die Übertragungscharakteristik des Schaltwandlers lautet

$$K\ \sim\ p$$

und die Ausgangsspannung

$$Um\ =\ K\ *\ Uq\ \sim\ p\ *\ Uq$$

Für K > 1 gilt Uq < Um und der rechte Zweig des Schaltwandlers muss getaktet werden, während der linke durchgeschaltet ist. Die Übertragungscharakteristik des Schaltwandlers lautet:

$$Um\ =\ Uq\ *\ 1\ /\ K$$

Daraus erkennt man, dass K nicht mehr proportional zum Tastverhältnis p ist. Der Verlauf ist nichtlinear. Bei p = 1 wäre die Ausgangsspannung theoretisch unendlich groß. In der Realität wäre sie 0, denn der rechte Anschluss der Spule wäre dauerhaft gegen Masse geschaltet. Durch den Einsatz einer programmierten Steuerlogik kann die Kennlinie der Schaltung linearisiert werden. Das Ergebnis wäre ein durch p linear einstellbarer Spannungswandler mit Transformator-Eigenschaften. In einem Ausführungsbeispiel mit maximalem Eingangsstrom von 3A und einer Eingangsspannung von max.15V ergibt sich eine Leistung von $P1\_max = 45W$. Diese Leistung deckt den Leistungsbedarf des Elektromotors (13) im stationären Betrieb. Ein schnelles Beschleunigen des Elektromotors (<500ms) ist jedoch nicht möglich. Als Beispiel ist die benötigte Rotationsenergie für einen Drehzahlsprung von n = 20000 1/min auf n = 60000 1/min berechnet zu ~ 18 W. Soll der Elektromotors den Drehzahlsprung in 200ms vollziehen, wird die Energie in einem Fünftel der Zeit benötigt. Somit verfünffacht sich die Leistung auf ~ 93 W.

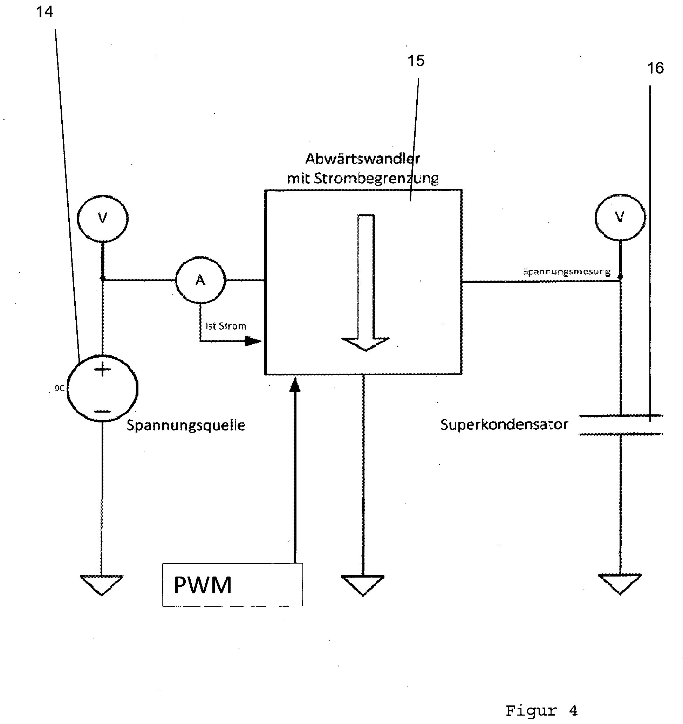

Die endstehende Stromspitze muss durch einen Zwischenspeicher gepuffert werden, damit die speisende Quelle nicht durch einen Überstrom überlastet wird. In Figur 4 ist ein beispielhafter Aufbau mit einem Superkondensator dargestellt. Der Superkondensator (16) übernimmt dann die Funktion der Spannungsquelle Uq (14). Bei einem Bremsvorgang mit Energierückgewinnung muss die Bremsenergie aufgefangen und gespeichert werden. Es muss deshalb ein Energiespeicher verwendet werden. Moderne Lithium Ionen Zellen sind in der Lage entsprechende Ströme abzugeben, allerdings sind sie für die permanente Ladungsverschiebung aus dem Speicher beziehungsweise in den Speicher, aus Lebensdauergründen, weniger geeignet. Dies dürfte sich allerdings in den nächsten Jahren ändern und Akku-Zellen dann auch geeignet. Erfindungsgemäß kann daher auch ein Akkumulator den Superkondensator ersetzen oder ergänzen. Den geeignetsten Speicher stellt heute ein Superkondensator dar. Als Superkondensator EDLC (electrochemical double layer capacitor) bezeichnet man eine Gruppe von Kondensatoren mit besonders hoher Energiedichte. Es gibt verschiedene Technologien, durch die ein Superkondensator realisiert werden kann, diese sind alle Bestandteile der vorliegenden Erfindung. Beschrieben wird hier der Doppelschichtkondensator. Doppelschichtkondensatoren ähneln mit ihrem Aufbau dem Elektrolytkondensator. Bei einer ausreichend großen Kapazität des Kondensators können starke Leistungsspitzen abgefangen werden. Eine besonders starke Bedämpfung des Eingangsstroms erhält man, wenn die Versorgungsquelle (14) durch eine entsprechende Schaltung (15) von dem Superkondensator entkoppelt wird. Dazu eignet sich eine Strombegrenzung, die dafür sorgt, dass der Superkondensator nur mit einem maximalen Strom nachgeladen werden kann. Eine geeignete Schaltung zur verlustarmen Strombegrenzung ist ein Schaltwandler (15) mit einer entsprechenden Regelung. Der Schaltwandler kann über einen Regler so betrieben werden, dass eine bestimmte Ausgangsspannung angestrebt wird. Beim Regelvorgang überprüft ein Vergleicher den aktuellen Ladestrom und korrigiert ggf. den Stellwert des Reglers. Wählt man einen Superkondensator mit der Nennspannung U_Kondensator = U_Betrieb, kann auf eine Spannungsregelung verzichtet werden. Bei Einsatz eines Abwärtswandlers kann die Kondensatorspannung durch den Ladevorgang nicht größer werden als die Betriebsspannung. Die Kondensatorspannung kann nur durch eine Energierückgewinnung größer als die Betriebsspannung werden. In diesem Fall sperrt der Laderegler, sodass kein Strom fließt solange die Spannung höher ist als die Betriebsspannung. Der Vorteil des Konzepts ist, dass nur eine Strombegrenzung realisiert werden muss.

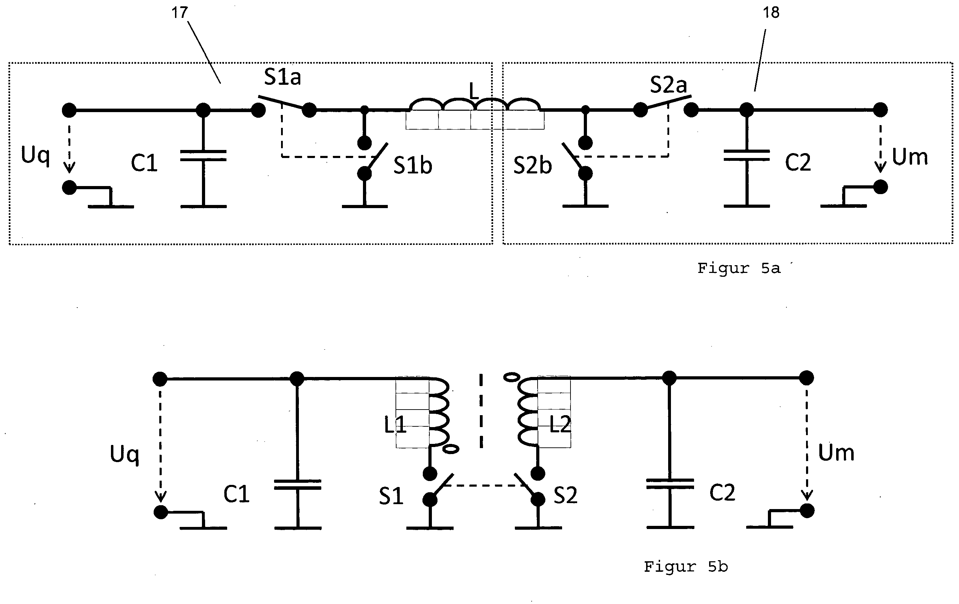

Der in den Figuren 5a und 5b dargestellte Auf- und Abwärtswandler (15a) (oder auch bidirektionale DC-/DC-Wandler) sind Ausführungsformen, des in Figur 3 dargestellten Prinzips. Sie zeichnet aus, dass der Wandler in beide Stromflussrichtungen als Auf- wie auch als Abwärtswandler betrieben wird.

Figur 5a: Die Schalter Sla und S1b arbeiten immer komplementär. Wird der Schalter Sla geschlossen, beginnt der Strom I_L über die Spule L aus dem Kondensator C1 zu fließen. Öffnet der Schalter, wird die Richtung des Stroms durch die Spule L aufrecht erhalten. Der Stromkreis ist über S1b geschlossen und der Strom fließt alleine durch die rechte Masche (18). Der Strom sinkt ab, bis er schließlich I_L = 0A durchschreitet und je nach Ladungszustand des Kondensators C2 negativ wird. Sobald der Schalter Sla wieder geschlossen wird, steigt der Strom als Folge wieder an. Der Schalter Sla wird über eine Ansteuerungselektronik geregelt oder gestellt. Mit einer PWM (Pulsweitenmodulation) oder einer PFM (Pulsfrequenzmodulation), die gegenphasig die Leistungsschalter Sla und S1b steuert, wird der mittlere Stromfluss durch die Spule L variiert. Somit wird das Strom- / Spannungs-übertragungsverhältnis eingestellt. Durch Vorgabe einer festen PWM auf einen der beiden Synchron-wandler lässt sich ein Strom- / Spannungsübersetzungsverhältnis einstellen. Durch die Kombination von Auf- und Abwärtswandler lässt sich das Übertragungsverhältnis sowohl kleiner als auch größer als Eins einstellen. Zur Übersicht sind in Tabelle 1 alle möglichen Betriebsarten des Wandlers aufgelistet.

Tabelle 1: Auflistung der möglichen Betriebsarten

| Ansteuerung von | Betriebsart |
|---|---|
| PWM S2a/S2b, Sla geschlossen | Aufwärtswandlung mit Stromfluss nach rechts oder Abwärtswandlung mit Stromfluss nach links |

(fortgesetzt)

| Ansteuerung von | Betriebsart |
|---|---|
| PWM S1a/S1b, S2a geschlossen | Abwärtswandlung mit Stromfluss nach links oder Aufwärtswandlung mit Stromfluss nach rechts |
| PWM S1a/S1b und inverses PWM S2a/S2b | Spezialfall: Inverswandlung |
| Sla und S2a geschlossen | Durchgeschalteter Betrieb |

Der Wandler (15a) eignet sich demnach für Systeme, die aus einer Quelle betrieben werden, aber auch in diese zurückspeisen sollen.

Durch die Verwendung eines Synchron-Wandlers wird ein bidirektionaler Stromfluss möglich. Der Wandler arbeitet als PWM-Steller und überträgt bei konstanter Einstellung Energie von der linken (17) zur rechten Seite (18) und umgekehrt. Die Funktionsweise kann als DC/DC-Transformator beschrieben werden, dessen Transformationsverhältnis durch das PWM-Verhältnis der aktiven Schaltelemente eingestellt werden kann. Das Transformationsverhältnis beschreibt hierbei im Wesentlichen das Spannungsverhältnis beider Seiten.

In Abbildung 5a muss bei Spannungen 1 < 2 die linke Seite (17) und bei Spannungen 1 > 2 die rechte Seite (18) getaktet werden. Bei der Schaltung gemäß Figur 5b wird immer nur das eine Schalterpaar S1 oder S2 getaktet, wobei dadurch die Spule (L1 L2) komplexer wird. Abhängig vom aktuellen Spannungszustand der beiden Seiten stellt sich die Stromflussrichtung ein.

In Figur 5b sind S1 und S2 ein Schalterpaar, welches immer komplementär aber prinzipiell wie Variante 5a arbeitet. Ein Vorteil dieses Wandlertyps ist die voll einstellbare Transformationseigenschaft durch eine PWM.

Ist nun die Spannungsversorgung Uq (linke Seite, 17) derart aufgebaut, dass sie als Spannungsquelle und Spannungssenke arbeiten kann, so kann der Gebläsemotor (13) (rechte Seite) beschleunigt (Spannungsversorgung = Quelle) oder abgebremst (Spannungsversorgung = Senke) werden. Die Beschleunigungs- und Bremsströme können bei typischer Beatmung hohe Werte erreichen, die sehr oft pro Minute ein Umschalten zwischen Quelle und Senke erfordern. Prinzipiell kann die Spannungsversorgung mit einem Akku realisiert werden, der mit jedem Zyklus entladen und geladen wird. Auch wenn die Ladungsmengen gering sind, wirken sich diese Zyklen negativ auf die Lebensdauer des Akkus aus. Besser ist es deshalb Kondensatoren und im Speziellen sogenannte DoppelSchicht-Kondensatoren (DLC) einzusetzen, die die Energie nicht in Form von chemischen Veränderungen sondern durch einfaches Polarisieren speichern. C1 wäre dann ein DLC, der aus einer Spannungsversorgung (14) (Netzteil, Akku o.ä.) geladen werden kann. Auf Grund der hohen Energiedichte in den DLCs können die Ladeströme so gestaltet werden, dass die Spannungsversorgung nur noch mit einem mittleren Strom belastet wird. So kann die Versorgung einfacher aufgebaut werden. Bei genügend großen DLCs ist sogar ein Wechseln des Akkus möglich, ohne dass die Beatmung einbricht oder angehalten werden muss.

Prinzipiell sind alle Gleichspannungswandler für diese Motoransteuerung denkbar, solange sie als Bidirektionale-Wandler aufgebaut sind. Es ist aber auch denkbar, unterschiedliche Wandler für das Beschleunigen und das Bremsen einzusetzen und diese entsprechend zu koppeln. Der Vorteil von Bidirektionale -Wandlern-Typen, die sowohl abwärts als auch aufwärts stellen können, ist, dass nicht zwischen Beschleunigen und Bremsen zu unterscheiden ist und somit eine Regelung deutlich vereinfacht wird.

Die Leistungsregelung für die Motorendstufe erfolgt über eine Treiberbrücke. Alternativ wird die Motorleistung durch die an die Motorendstufe angelegte Spannung eingestellt bzw. geregelt. Ein Vorteil ist, dass die Kombination aus Motorendstufe und Motor als eigenständige Einheit betrachtet werden kann. Die Kombination verhält sich an einer Spannungsquelle wie ein einfacher einphasiger Gleichstrommotor.

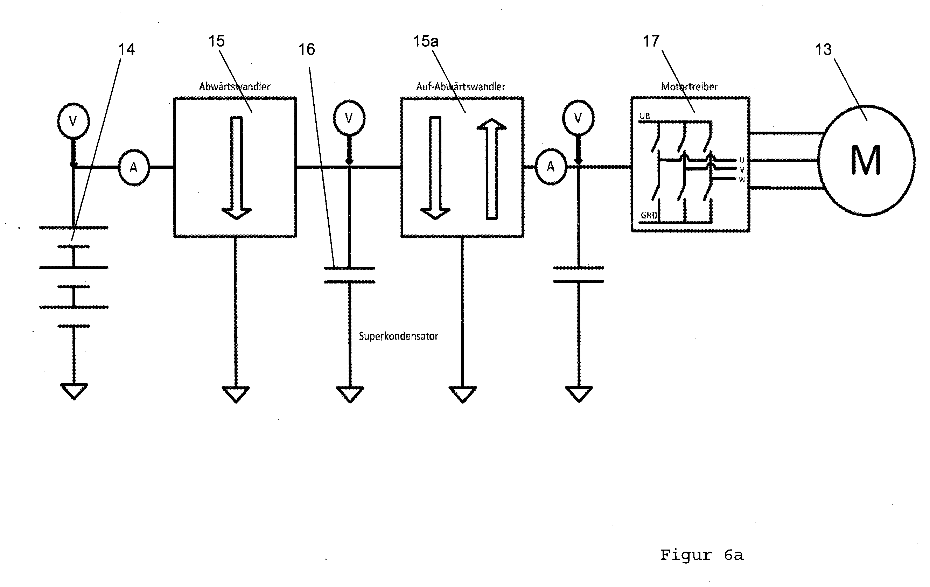

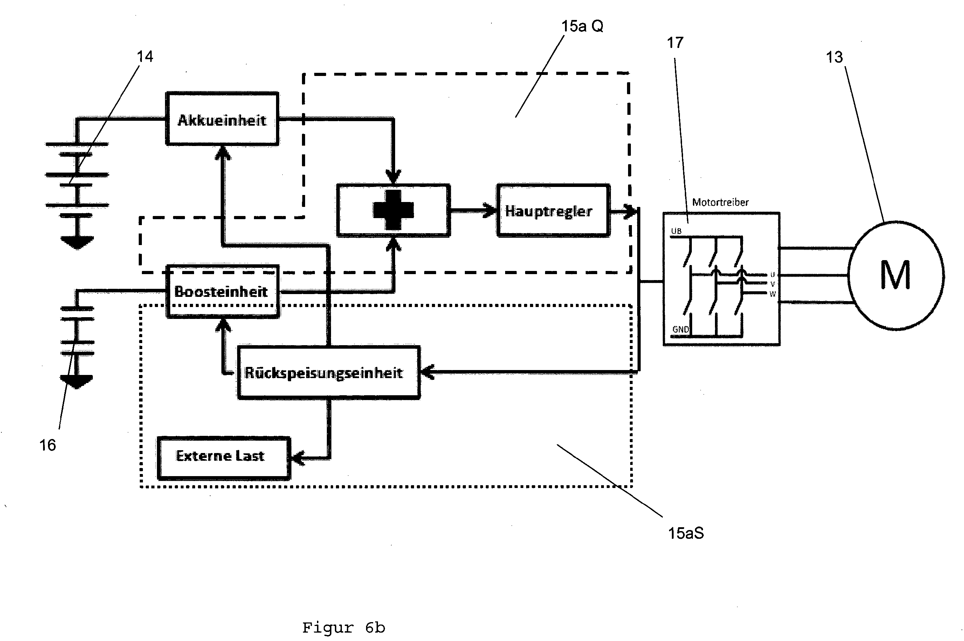

Aus den in den Figuren 2 bis 5 dargestellten Systemblöcken kann ein Gesamtsystem gemäß den Figuren 6 entworfen werden. In Fig. 6a ist eine sequenzielle Struktur dargestellt, bei der zuerst der eingezeichnete Superkondensator (C1) (16) in einer Sequenz geladen werden muss. Anschließend kann der synchrone Auf- und Abwärtswandler (15a) aus der Kondensatorspannung gespeist werden. Während des Betriebs wird der Kondensator stets mit Maximalstrom (3A) nachgeladen, wenn seine Spannung kleiner ist als die Betriebsspannung am Eingang des Gesamtkonzepts. Über den synchronen Auf- Abwärtswandler (15a) kann ein Übertragungsverhältnis zwischen Kondensatorspannung und Motortreiberspannung eingestellt werden. Dadurch wird die Motorleistung variiert und die Stromflussrichtung gesteuert. Die Leistung von Ein- und Ausgang der Schaltung sind über einen langen Zeitraum betrachtet immer gleich. Es können jedoch hohe Spitzenleistungen am Ausgang abgegeben werden. Dieses Verhalten ist ideal, wenn ein Motor betrieben wird. Betrachtet man das Konzept von der Motorseite aus, ist es bei überschüssiger Drehzahl möglich, den Motor als Generator zu betreiben und Energie in den Superkondensator (16) zu speichern. Die daraufhin im Kondensator gespeicherte Energie kann für die nachfolgende Beschleunigung genutzt werden. Als Folge sinkt der mittlere Betriebsstrom und somit die aufgenommene Leistung.

Das Gesamtsystem kann gemäß Figur 6b auch als parallele Struktur aufgebaut werden. Hierbei ist parallele Energieversorgung des Motors über die primäre Energiequelle (14) und die sekundäre Energiequelle (16) (Superkondensator) vorgesehen. Über den oberen Pfad (15aQ), hier über eine gestrichelte Umrahmung gekennzeichnet, wird der Motor aus der primären Energiequelle (14) mit Spannung versorgt.

Über den unteren Pfad (15aS), hier über eine gepunktete Umrahmung gekennzeichnet, wird Bremsenergie des Motors im Generatorbetrieb in dem Kondensator (16) gespeichert. Die im Kondesator (16) gespeicherte Energie kann dann über die Boosteinheit wieder für die Energieversorgung des Motors verwendet werden.

Der obere Pfad (15aQ) und der untere Pfad (15aS) übernehmen somit die Funktionen des Auf-Abwärtswandlers aus Figur 6a.

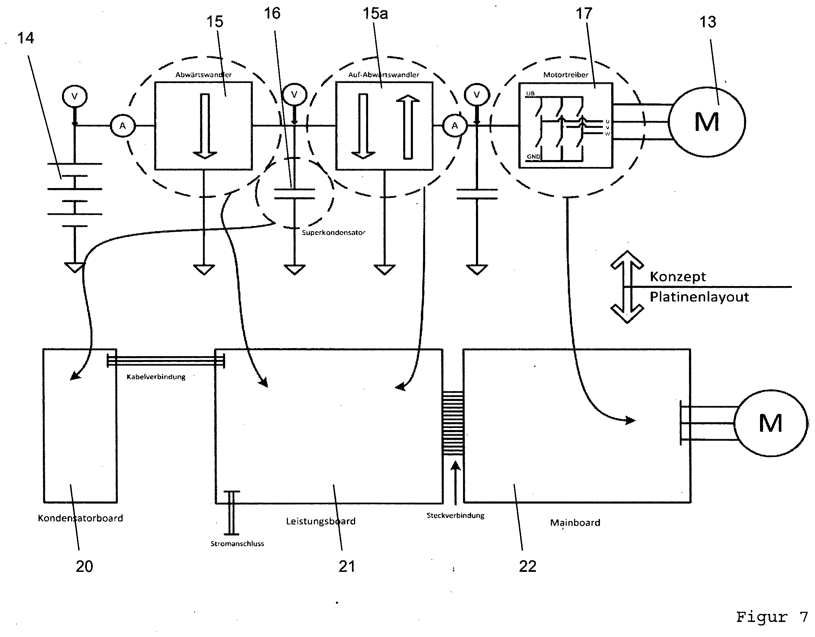

Um die Realisierung des Antriebskonzepts modular zu gestalten, wird das Gesamtkonzept - wie in Figur 7 dargestellt - in 3 Baugruppen unterteilt, die wiederum auf eigenen Platinen realisiert werden. Sie lauten

1. Mainboard (22)
2. Leistungsboard (21)
3. Kondensatorboard (20)

Die Platinen Leistungsboard (21) und Mainboard (22) können über einen Steckersystem verbunden werden. Das Kondensatorboard (20) kann über eine Kabelverbindung mit dem Leistungsboard (21) verbunden werden. Durch die modulare Bauweise ist das Antriebskonzept flexibel gegenüber Neuentwicklungen. Die modulare Bauweise ermöglicht beispielsweise, Leistungsboard (21) und Mainboard (22) mit unterschiedlichen Kondensatorboards (20) zu verwenden. Da das entwickelte Blockschaltbild aus Figur 6 keine Bausteine für Peripheriezwecke und zur Steuerung oder Regelung enthält, ist es erforderlich weitere Komponenten hinzuzufügen. Um einen Überblick zu vermitteln, welche Komponenten zusätzlich hinzugefügt wurden, werden die drei Platinen kurz erläutert.

Das Mainboard (22) stellt den zentralen Knotenpunkt aller Datenleitungen dar. Es beinhaltet folgende Komponenten:

• Mikrocontroller

• Motortreiber

• Peripherie zur Kommunikation

• Netzteil

• Überspannungsschutz

Auf dem Mainboard wird ein 32-Bit Mikrocontroller verwendet, welcher durch eine Programmierung die zentrale Steuer- und Regeleinheit des Antriebskonzepts darstellt. Hier befindet sich eine Motorendstufe zur Ausführung der Motorkommutierung. Zudem sind verschiedene Peripherieelemente vorgesehen, die die Kommunikation mit dem Leistungsboard, wie auch mit dem Anwender ermöglichen. Um die erforderlichen Kleinspannungen für den Prozessor und Peripherie zu erzeugen, befindet sich ein Netzteil auf dem Mainboard.

Auf dem Leistungsboard (21) ist die Leitungselektronik für die Energieversorgung des Motortreibers realisiert. Die Systemblöcke, die hier realisiert wurden, sind:

• Synchroner Auf- und Abwärtswandler zur Motorversorgung Die Schaltfrequenz des Wandlers liegt bei 100kHz. Um die Ausgangsspannung des Auf- und Abwärtswandlers zu messen, wurde ein Messverstärker vorgesehen.

• Abwärtswandler zum Laden des Superkondensators Die Schaltfrequenz des Wandlers wurde auf 100kHz festgelegt. Die im Konzept vor dem Kondensatorladeregler eingezeichnete Strommessung wurde durch einen Halleffektsensor realisiert. Es wurde eine Spannungsmessung am Eingang des Ladereglers und am Superkondensator vorgesehen.

• Vorspannungsgenerator Über eine Zenerdiodenschaltung wird die Kapazität der Ladungspumpe des jeweiligen Gatetreibers 10V über die aktuelle Sourcespannung aufgeladen.

• Bremsstufe - Motorbremsung ohne Energierückgewinnung Bei einer Bremsung über die Bremsstufe wird die Rotationsenergie in einem Leistungswiderstand in Wärme umgewandelt.

- Überspannungsschutz

- PWM-Generatoren

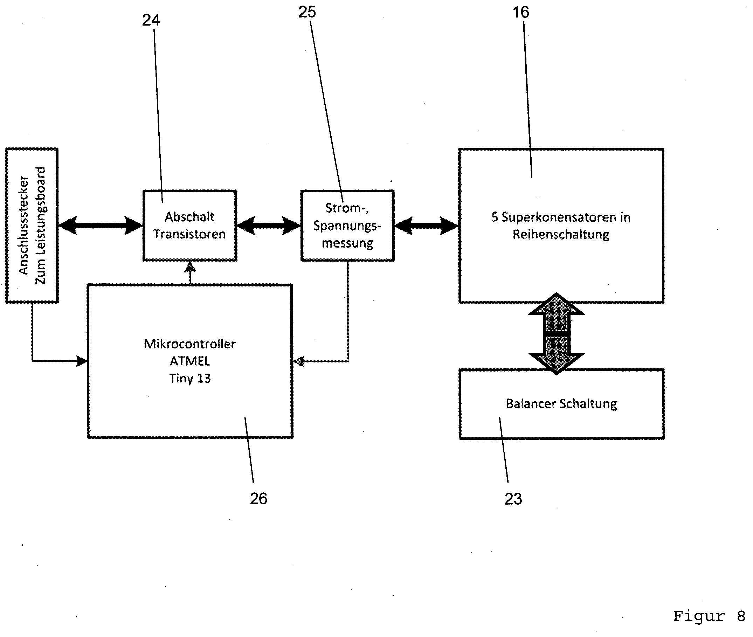

Auf dem in Figur 8 dargestellten Kondensatorboard (20) sind 5 Superkondensatoren verbaut. Zudem ist eine Sicherheitsschaltung realisiert worden, die eine Überspannung der Kondensatorzellen verhindert. Das Board enthält folgende Baugruppen:

- Kondensatoren Als Energiespeicher wurde eine Reihenschaltung von 5 Superkondensatoren (16) der Firma Nesscap verwendet. Die Kondensatorbatterie bietet somit eine Kapazität von 2F bei einer Betriebsspannung von 13,5V.
- Balancerschaltung (23) zur Zellspannungsüberwachung Die Schutzbeschaltung wurde durch eine NPN Leistungstransistorschaltung realisiert, die jeden Kondensator bei einer Überspannung überbrücken kann. Die Schaltung wandelt überschüssige Ladung in Wärme um.
- Abschalttransistoren (24) Es wurde eine MOSFET-Schaltung aus zwei antiparallelen P Kanal MOSFETs eingesetzt, um die Verbindung zwischen den Kondensatoren und dem Leistungsboard trennen zu können. Die MOSFETS können durch den auf der Platine befindlichen Mikrocontroller ein- und abgeschaltet werden. Durch den Einsatz der Transistoren erhöht sich der Innenwiderstand der Schaltung um die Summe der Transistorwiderstände RDSon (2 mal ca. 5 mOhm).
- Messelektronik (25) Das Kondensatorboard verfügt über eine Strom- und Spannungsmessung, mit der eine Überlastung und der aktuelle Betriebszustand des Superkondensators ermittelt werden kann.
- 8-Bit Mikrocontroller (26) Für die Auswertung der Messwerte wird ein Mikrocontroller vom Typ Tiny13 der Firma ATMEL verwendet.

Zur Überprüfung der Energierückgewinnung wurde das Gebläse mit einem realistischen Beatmungsmuster betrieben.

Tabelle 2: Einstellungen für Messung

| |
|---|
| Beatmungsfrequenz 30/min |
| Startwert 0% |
| Anstiegszeit 100 ms |
| Endwert 90% |
| Abfallzeit 280ms |
| I:E 35% |

Es wurden zwei je 10 minütige Vergleichsmessungen (A: Frequenz 30/min; B: Frequenz 60/min) durchgeführt, bei denen der Bremsbetrieb zwischen

1. Bremsung über Energierückgewinnung
2. Bremsung über Bremsstufe variiert wurde.

Die Differenz der Energie ist dann ein Maß für die Energieeffizienz des Konzepts mit Energierückgewinnung.

**A:** Aufgenommene Energie während der ersten Messung (Frequenz 30/min)

- ohne Energierückgewinnung 8,6 kWs

- mit Energierückgewinnung 7,0 kWs

Die Differenz der Energiemengen beträgt 1,6 kWs, was einer Energieersparnis von 19% entspricht.
B: Aufgenommene Energie während der ersten Messung (Frequenz 60/min)

- ohne Energierückgewinnung 9,0 kWs

- mit Energierückgewinnung 7,1 kWs

Die Energieersparnis nimmt bei einer höher eingestellten Frequenz von 19% auf 21% leicht zu. Somit ist eine kleine Abhängigkeit der Frequenz zur Menge der rückgewonnenen Energie vorhanden. Die beim Betrieb festgestellte Energieersparnis von rund 20% ermöglicht eine dementsprechend längere Betriebszeit des Beatmungsgeräts. Anderseits ist es durch die verbesserte Technik nun möglich, kleinere Geräte mit kleineren Akkupacks und gleicher Laufzeit zu bauen.

**Patentansprüche**

1. Beatmungsgerät (1) mit einem Elektromotor (13) und zumindest einer primären Energiequelle (14) für den Elektromotor (13) und einer sekundären Energiequelle (16), wobei die sekundäre Energiequelle als Spannungssenke für Generatorstrom vom Elektromotor (13) ausgebildet ist, **dadurch gekennzeichnet, dass** der Generatorstrom durch eine Transformation von kinetischer Energie des Elektromotors (13) erzeugt wird, und dass die Transformation nach einem Ende einer Inspirationsphase und vor einem Beginn einer Exspiration erfolgt und dass eine Sensoreinrichtung / Analyseeinheit zur Steuerung und Regelung des Beatmungsbetriebes verwendet wird, wobei zur Erfassung eines Druckes ein Druckmessssschlauch (6) verwendet wird und wobei der Betrieb des Elektromotors (13) über eine Motorsteuerung geregelt wird und wobei die Sensoreinrichtung mindestens ein mit einem Atemgasstrom im Zusammenhang stehende Signal ermittelt und der Analysator aus diesem Signal die Inspirationsphasen und die Exspirationsphasen erkennt, wobei durch eine Drehzahländerung des Elektromotors (13) der Druck eingestellt wird.

2. Beatmungsgerät nach Anspruch 1 **dadurch gekennzeichnet, dass** die sekundäre Energiequelle als Kondensator (16) ausgebildet ist

3. Beatmungsgerät nach zumindest einem der vorhergehenden Ansprüche **dadurch gekennzeichnet, dass** zwischen Elektromotor (13) und der Energiequelle (14, 16) Mittel (15, 15a) angeordnet sind, die einen bidirektionalen Stromfluss ermöglichen.

4. Beatmungsgerät nach zumindest einem der vorhergehenden Ansprüche **dadurch gekennzeichnet, dass** zwischen Elektromotor (13) und der Energiequelle (14,16) zumindest ein Wandler (15, 15a) angeordnet ist, der Spannung und/oder Strom wandelt.

5. Beatmungsgerät nach zumindest einem der vorhergehenden Ansprüche **dadurch gekennzeichnet, dass** der Wandler (15, 15a) unidirektional ist.

6. Beatmungsgerät nach zumindest einem der vorhergehenden Ansprüche **dadurch gekennzeichnet, dass** zwei unidirektionale Wandler (15, 15a) mit entgegengesetzter Richtung für Spannung und/oder Strom vorhanden sind, einer für die Senken-Richtung, einer für Quellen-Richtung.

7. Beatmungsgerät nach zumindest einem der vorhergehenden Ansprüche **dadurch gekennzeichnet, dass** ein bidirektionaler Wandler (15, 15a) vorhanden ist.

8. Beatmungsgerät nach zumindest einem der vorhergehenden Ansprüche **dadurch gekennzeichnet, dass** ein bidirektionaler Stromfluss zwischen Motor (13) und der Energiequelle (14,16) durch Verwendung eines DC/DC-Synchron-Wandlers (15a) realisiert ist.

9. Beatmungsgerät nach Anspruch 8 **dadurch gekennzeichnet, dass** der **DC/DC**-Synchron-Wandler (15a) zumindest ein Schalter-paar (SI / S2) aufweist

10. Beatmungsgerät nach Anspruch 8 **dadurch gekennzeichnet, dass** der **DC/DC**-Synchron-Wandler (15a) zumindest zwei Schalterpaare (SIa / SIb, S2a / S2b) aufweist.

11. Verfahren zur Nutzung von Bremsenergie bei einem Beatmungsgerät (1) nach einem der vorhergehenden Ansprüche, **dadurch gekennzeichnet, dass** der Generatorstrom durch eine Transformation von kinetischer Energie des Elektromotors (13) erzeugt wird und die Transformation nach einem Ende einer Inspirationsphase und vor einem Beginn einer Exspiration erfolgt.

**12.** Verfahren nach Anspruch 11 **dadurch gekennzeichnet, dass** ein bidirektionaler Stromfluss zwischen Motor (13) und der Energiequelle (14, 16) durch Verwendung zumindest eines Wandlers (15, 15a) realisiert ist.

**13.** Verfahren nach Anspruch 11 oder 12 **dadurch gekennzeichnet, dass** das Übertragungsverhältnis von Strom- oder Spannungsfluss zwischen Motor (13) und der Energiequelle (14,16) durch Pulsweitenmodulation oder Pulsfrequenz-modulation der Leistungsschalter des Wandlers (15, 15a)erfolgt.

**14.** Verfahren nach zumindest einem der Ansprüche 11 bis 13 dadurch ge-kennzeichnet, dass der Elektromotor (13) im Generatorbetrieb Spannung erzeugt, die in einem Kondensator oder Akkumulator gespeichert wird und die Spannung danach zumindest teilweise wieder für die Energieversorgung des Elektromotors genutzt wird.

**Claims**

**1.** Ventilator (1) having an electric motor (13) and at least one primary energy source (14) for the electric motor (13) and a secondary energy source (16), wherein the secondary energy source is embodied as a voltage sink for generator current from the electric motor (13), **characterized in that** the generator current is produced by transforming kinetic energy of the electric motor (13) and **in that** the transformation is effectuated after an end of an inspiration phase and before the start of an expiration and **in that** a sensor device/analysis unit is used for controlling and regulating the ventilation operation, wherein a pressure measuring tube (6) is used to capture a pressure and wherein the operation of the electric motor (13) is regulated by way of a motor controller and wherein the sensor device ascertains at least one signal that is related to a respiratory gas flow and the analyser identifies the inspiration phases and the expiration phases from this signal, wherein the pressure is set by a change in speed of the electric motor (13).

**2.** Ventilator according to Claim 1, **characterized in that** the secondary energy source is embodied as a capacitor (16).

**3.** Ventilator according to at least one of the preceding claims, **characterized in that** means (15, 15a) which facilitate a bidirectional current flow are arranged between the electric motor (13) and the energy source (14, 16).

**4.** Ventilator according to at least one of the preceding claims, **characterized in that** at least one converter (15, 15a) which converts a voltage and/or current is arranged between the electric motor (13) and the energy source (14, 16).

**5.** Ventilator according to at least one of the preceding claims, **characterized in that** the converter (15, 15a) is of a unidirectional type.

**6.** Ventilator according to at least one of the preceding claims, **characterized in that** two unidirectional converters (15, 15a) with the opposite direction for the voltage and/or current are present, one for the sink direction and one for the source direction.

**7.** Ventilator according to at least one of the preceding claims, **characterized in that** a bidirectional converter (15, 15a) is present.

**8.** Ventilator according to at least one of the preceding claims, **characterized in that** a bidirectional current flow is realized between motor (13) and the energy source (14, 16) by using a DC/DC synchronous converter (15a).

**9.** Ventilator according to Claim 8, **characterized in that** the DC/DC synchronous converter (15a) has at least one switch pair (S1/S2).

**10.** Ventilator according to Claim 8, **characterized in that** the DC/DC synchronous converter (15a) has at least two switch pairs (S1a/S1b, S2a/S2b).

**11.** Method for using braking power in a ventilator (1) according to any one of the preceding claims, **characterized in that** the generator current is produced by transforming kinetic energy of the electric motor (13) and the transformation is effectuated after an end of an inspiration phase and before the start of an expiration.

**12.** Method according to Claim 11, **characterized in that** a bidirectional current flow between motor (13) and the energy source (14, 16) is realized by using at least one converter (15, 15a).

**13.** Method according to Claim 11 or 12, **characterized in that** the transfer ratio of current or voltage flow between motor (13) and the energy source (14, 16) is effectuated by pulse-width modulation or pulse-frequency modulation of the circuit breakers of the converter (15, 15a).

**14.** Method according to at least one of claims 11 to 13, **characterized in that** the electric motor (13) produces a voltage during the generator operation, said voltage being stored in a capacitor or accumulator, and the voltage is subsequently used again, at least in part, for supplying power to the electric motor.

## Revendications

**1.** Appareil respiratoire (1) comprenant un moteur électrique (13) et au moins une source d'énergie primaire (14) pour le moteur électrique (13) et une source d'énergie secondaire (16), la source d'énergie secondaire étant réalisée sous la forme d'un puits de tension pour le courant de générateur du moteur électrique (13), **caractérisé en ce que** le courant de générateur est généré par une transformation de l'énergie cinétique du moteur électrique (13), et **en ce que** la transformation a lieu après une fin d'une phase d'inspiration et avant un début d'une expiration et **en ce qu'**un dispositif de détection / une unité d'analyse est utilisé(e) pour la commande et la régulation du régime de respiration,
un tuyau de mesure de pression (6) étant utilisé pour acquérir une pression et le fonctionnement du moteur électrique (13) étant régulé par le biais d'une commande de moteur
et le dispositif de détection déterminant au moins un signal qui est en relation avec un flux de gaz respiratoire et l'analyseur reconnaissant les phases d'inspiration et les phases d'expiration à partir de ce signal, la pression étant réglée par une modification de la vitesse de rotation du moteur électrique (13).

**2.** Appareil respiratoire selon la revendication 1, **caractérisé en ce que** la source d'énergie secondaire est réalisée sous la forme d'un condensateur (16).

**3.** Appareil respiratoire selon au moins l'une des revendications précédentes, **caractérisé en ce qu'**entre le moteur électrique (13) et la source d'énergie (14, 16) sont disposés des moyens (15, 15a) qui rendent possible un flux de courant bidirectionnel.

**4.** Appareil respiratoire selon au moins l'une des revendications précédentes, **caractérisé en ce qu'**entre le moteur électrique (13) et la source d'énergie (14, 16) est disposé au moins un convertisseur (15, 15a) qui convertit la tension et/ou le courant.

**5.** Appareil respiratoire selon au moins l'une des revendications précédentes, **caractérisé en ce que** le convertisseur (15, 15a) est unidirectionnel.

**6.** Appareil respiratoire selon au moins l'une des revendications précédentes, **caractérisé en ce que** deux convertisseurs (15, 15a) unidirectionnels sont présents, ayant des directions opposées pour la tension et/ou le courant, un pour la direction du puits, un pour la direction de la source.

**7.** Appareil respiratoire selon au moins l'une des revendications précédentes, **caractérisé en ce qu'**un convertisseur (15, 15a) bidirectionnel est présent.

**8.** Appareil respiratoire selon au moins l'une des revendications précédentes, **caractérisé en ce qu'**un flux de courant bidirectionnel est réalisé entre le moteur (13) et la source d'énergie (14, 16) en utilisant un convertisseur synchrone CC/CC (15a).

**9.** Appareil respiratoire selon la revendication 8, **caractérisé en ce que** le convertisseur synchrone CC/CC (15a) possède au moins une paire de commutateurs (S1, S2).

**10.** Appareil respiratoire selon la revendication 8, **caractérisé en ce que** le convertisseur synchrone CC/CC (15a) possède au moins deux paires de commutateurs (S1a/S1b, S2a/S2b).

**11.** Procédé d'utilisation de l'énergie de freinage avec un appareil respiratoire (1) selon l'une des revendications précédentes, **caractérisé en ce que** le courant de générateur est généré par une transformation de l'énergie cinétique du moteur électrique (13), et la transformation a lieu après une fin d'une phase d'inspiration et avant un début d'une expiration.

**12.** Procédé selon la revendication 11, **caractérisé en ce qu'**un flux de courant bidirectionnel est réalisé entre le moteur (13) et la source d'énergie (14, 16) en utilisant au moins un convertisseur (15, 15a).

**13.** Procédé selon la revendication 11 ou 12, **caractérisé en ce que** le rapport de transmission du flux de courant ou de tension entre le moteur (13) et la source d'énergie (14, 16) est réalisé par modulation d'impulsions en largeur ou modulation de fréquence d'impulsions du commutateur de puissance du convertisseur (15, 15a).

**14.** Procédé selon au moins l'une des revendications 11 à 13, **caractérisé en ce que** le moteur électrique (13) en régime générateur génère une tension qui est accumulée dans un condensateur ou un accumulateur et la tension est ensuite au moins partiellement réutilisée pour l'alimentation en énergie du moteur électrique.

Figur 1

14     15     13

I1     I2

Uq

Um

M

Übertragungsverhältnis

Figur 2

Figur 3

14

15

16

Abwärtswandler
mit Strombegrenzung

V

A

Ist Strom

Spanrungsmesung

V

DC

+

−

Spannungsquelle

Superkondensator

PWM

Figur 4

Figur 5a

Figur 5b

Figur 6a

Figur 6b

EP 2 612 688 B1

Figur 7

24 25 16

| Anschlussstecker Zum Leistungsboard | → Abschalt Transistoren ← | → Strom-, Spannungs- messung ← | → 5 Superkonensatoren in Reihenschaltung |

Mikrocontroller
ATMEL
Tiny 13

Balancer Schaltung

26 23

Figur 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2010170513 A1